# EUROPEAN PATENT SPECIFICATION

(11) **EP 1 716 810 B1**
(45) Date of publication and mention of the grant of the patent: **15.10.2008**
(21) Application number: 06008448.0
(22) Date of filing: 24.04.2006
(51) Int. Cl.: A61B 17/04, A61B 17/28

(54) **Endoscopic surgical instrument**
Endoskopisches chirurgisches Instrument
Instrument chirurgical endoscopique

(30) Priority: 26.04.2005 JP 2005128067; 26.01.2006 JP 2006017039
(43) Date of publication of application: 02.11.2006
(73) Proprietor: Niti-on Co., Ltd., Funabashi-shi, Chiba-ken (JP)
(72) Inventor: Uchida, Kazunori, Hiroshima-shi Hiroshima-ken (JP)
(74) Representative: Goddar, Heinz J.

(56) References cited:
- DE-A1- 4 314 463
- DE-C1- 4 218 191
- FR-A- 2 699 394
- US-A1- 2005 059 983

## Description

### Field of the Invention

The present invention relates to an endoscopic surgical instrument, and particularly, the present invention relates to a needle holder provided with a needle holding part at a front end of an insertion part into an abdominal cavity.

### Description of the related art

A surgery is basically composed of ablation, dissection, and a suture ligation, and a suture ligation craft is also an essential art in an endoscopic surgery. Conventionally, in the case of performing the suture ligation by a needle holder in the endoscopic surgery, an external ligation for connecting a suture thread outside the body, feeding it in the abdominal cavity, and completing the ligation; and an internal ligation for performing the ligation by a forceps in the abdominal cavity have been implemented.

The external ligation is a simple method, however, a special device is necessary for an instrument for use in a ligation.

In the external ligation, there is tension on the suture tissue because the ligation part formed outside the body is inserted into the abdominal cavity, so that there is a danger of damaging the tissue. Therefore, it can be said that the ideal ligation is the internal ligation. However, the internal ligation is the craft demanding a high technology because a three-dimensional forceps behavior should be recognized on a two-dimensional monitor and the ligation is performed in a limited space, namely, the abdominal cavity.

When performing the ligation inside the body, it is difficult to control a suture thread using the forceps in the limited space and this behavior should be performed on the two-dimensional monitor in which perspective is hardly recognized. In order to solve this problem, a device is necessary for the instrument used for the internal suture ligation.

An endoscopic surgical instrument with the features of the preamble of claim 1 is known from FR-A-2699 394. Reference is made to DE 42 18 191 C1 and DE 42 14 463 A1.

In the case of performing the ligation in the abdominal cavity, conventionally, in place of the needle holder, a scissor is inserted in the abdominal cavity to cut the extra suture thread. Therefore, in order to complete the suture, the complicated task of interchanging the instrument from the needle holder into the scissors is needed.

If a function to cut the thread is added to the needle holder, this complicated task is made unnecessary and a danger of damaging the organs due to interchanging of the forceps is decreased. However, in the forceps of performing the suture ligation regardless of the needle holder and an auxiliary forceps, there is a danger of cutting the thread by mistake upon the ligation when a needle grasping face and a thread cutting function are added to this needle holder and there is a danger in cutting the thread when shifting the thread in the abdominal cavity from one arm to the other because the forceps of performing the suture ligation is controlled with the thread grasped. Particularly, when shifting the thread in the abdominal cavity, there is a great danger that the needle drops and is missed in the abdominal cavity. Accordingly, the thread cutting function should be separated from the needle holding part and the needle grasping face.

### SUMMARY OF THE INVENTION

The present invention has been made taking the foregoing problems into consideration and an object of which is to provide an endoscopic surgical instrument having a needle holder provided with a needle holding part at a front end of an insertion part into an abdominal cavity.

In order to attain the above-described object, according to the present invention, in the endoscopic surgical instrument with the features of first part of claim 1.

A contact part of the movable jaw and the tubular shaft functions as a scissors so that the suture thread is cut between the thread locking part and the tubular shaft when the movable jaw is closed.

Further, by providing an enough gap for the suture thread to freely move between the thread locking part and a tubular shaft, the thread hitched to the thread locking part can be removed from the thread locking part even if a movable jaw is closed after the ligation.

Alternatively, when the movable jaw is closed, the suture thread is prevented from being cut between the thread locking part and the tubular shaft, and this makes it possible to cut the extra suture thread without shifting the instrument from one arm to the other upon the ligation.

### BRIEF DESCRIPTION OF THE DRAWINGS

Fig. 1 is a front view of an endoscopic surgical instrument according to the present invention;
Fig. 2 is an enlarged view of a needle holding part with a movable jaw closed;
Fig. 3 is an enlarged view of the needle holding part with a movable jaw opened;
Fig. 4 is an enlarged bottom view of the needle holding part;
Fig. 5 is a sectional view taken on a line A-A in Fig. 4;
Fig. 6 is an enlarged front view of the needle holding part according to the other example of the endoscopic surgical instrument according to the present invention;
Fig. 7 is a bottom view of Fig. 6;
Fig. 8 is an explanatory view showing the state that a suture thread is locked by a thread locking part; and
Fig. 9 is an explanatory view showing the state that the suture thread is cut by a scissors composed of the thread locking part and a tubular shaft.

### DETAILED DESCRIPTION OF THE INVENTION

Providing a needle holding part composed of a movable jaw and a fixed jaw at a front end of a tubular shaft, the movable jaw is disposed to be capable of rotating around the fixed jaw through a pivot shaft due to the remote control using a handle. Then, a thread locking part is formed at the opposite side across the pivot shaft of the movable jaw, and when the movable jaw is opened, the thread locking part slightly protrudes outside a tubular shaft and a suture thread is allowed to be hitched there.

In addition, when the movable jaw is closed, the thread locking part is prevented from protruding outside the tubular shaft, and providing an enough gap for the suture thread to freely move between the thread locking part and the tubular shaft, it is possible to certainly remove the suture thread from the thread locking part after the ligation.

A contact part of the movable jaw and the tubular shaft functions as a scissors so that the suture thread is cut between the thread locking part and the tubular shaft when the movable jaw is closed.

### (First embodiment)

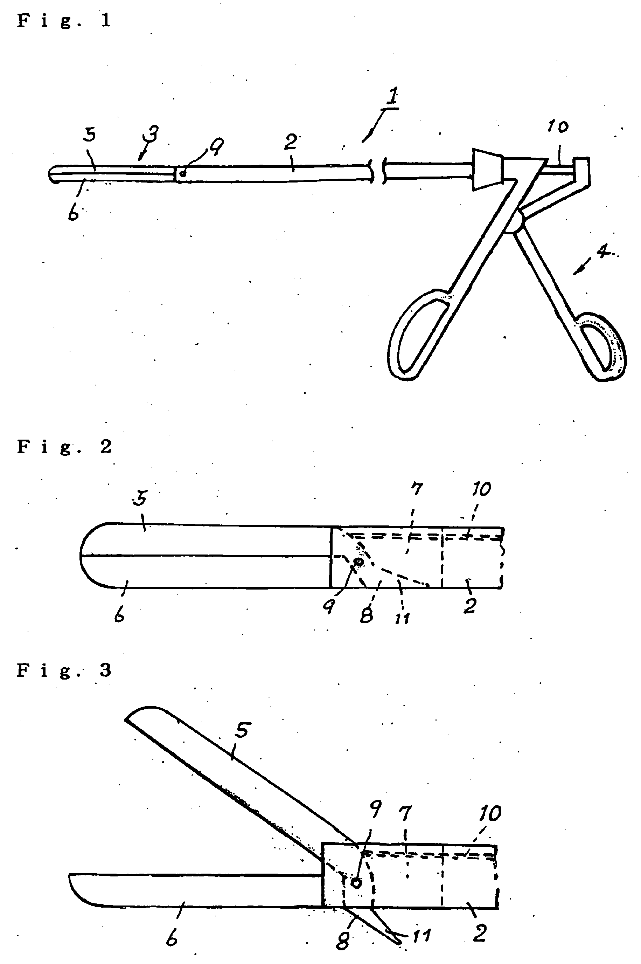

Next, an embodiment of an endoscopic surgical instrument will be described taking the case that a thread cutting function is not provided with reference to Figs. 1 to 5. In the drawings, a reference numeral 1 denotes an endoscopic surgical instrument. A needle holding part 3 is disposed at its front end across a tubular shaft 2 that is an insertion part and an operation handle 4 is disposed at hand.
The tubular shaft 2 is configured by a movable jaw 5 an a fixed jaw 6, and on a contact face of the movable jaw 5 and the fixed jaw 6, a concave-convex groove for holding the needle or the like is formed, respectively. Then, the fixed jaw 6 is fixed to the tubular shaft 2. Further, the fixed jaw 6 may be integrated with the fixed jaw 6 functionally, or they may be integrated physically. In other words, the fixed jaw 6 may function as the needle holding part 2 with respect to the movable jaw 5.

On the center part of the front side of the tubular shaft 2, a U-shaped space 7 is formed vertically, and an axis support part 8 can move in this space 7, which is narrowly elongated toward the side of the handle 4 of the movable jaw 5. Then, the movable jaw 5 is supported by the axis so as to be capable of rotating by this axis support part 8. A reference numeral 10 denotes a wire for opening and closing the movable jaw 5, which is connected slightly above the axis support part 8 of the movable jaw 5 and of which other end is coupled to the handle 4.

At the front end of the axis support part 8 of the movable jaw 5, a thread locking part 11 is provided. When the movable jaw 5 is opened, the thread locking part 11 protrudes outside the tubular shaft 2 so that the suture thread is hitched there. In addition, when the movable jaw 5 is closed, the thread locking part 11 is prevented from protruding outside the tubular shaft 2. In order to smoothly move this instrument into or out of the inside of a human body and prevent to damage the body by the thread locking part 11 without paying attention, the thread locking part 11 is protruded from the tubular shaft 2 only when needed.

A reference numeral 12 denotes a thread pulling out gap, and this is an enough gap for the suture thread to freely move between the thread locking part 11 and the tubular shaft 2 with the movable jaw 5 closed. This gap is provided so that the suture thread can be removed between the tubular shaft 2 and the thread locking part 11 as the movable jaw 5 is closed after the ligation is completed.

Next, a method of using the endoscopic surgical instrument will be described. A needle with a thread is clipped between the movable jaw 5 and the fixed jaw 6 of the needle holding part 3 at the front end of the tubular shaft 2 so as to suture a desired part. Then, after completion of suture, the suture thread is hitched to the thread locking part 11 and the suture thread is ligated by pulling the endoscopic surgical instrument 1 outside the body. When a knot is formed, the endoscopic surgical instrument 1 is pushed inside the body so as to pull the suture thread out from the thread locking part 11. Then, at a new suture part, the same operation is repeated.

### (Second Embodiment)

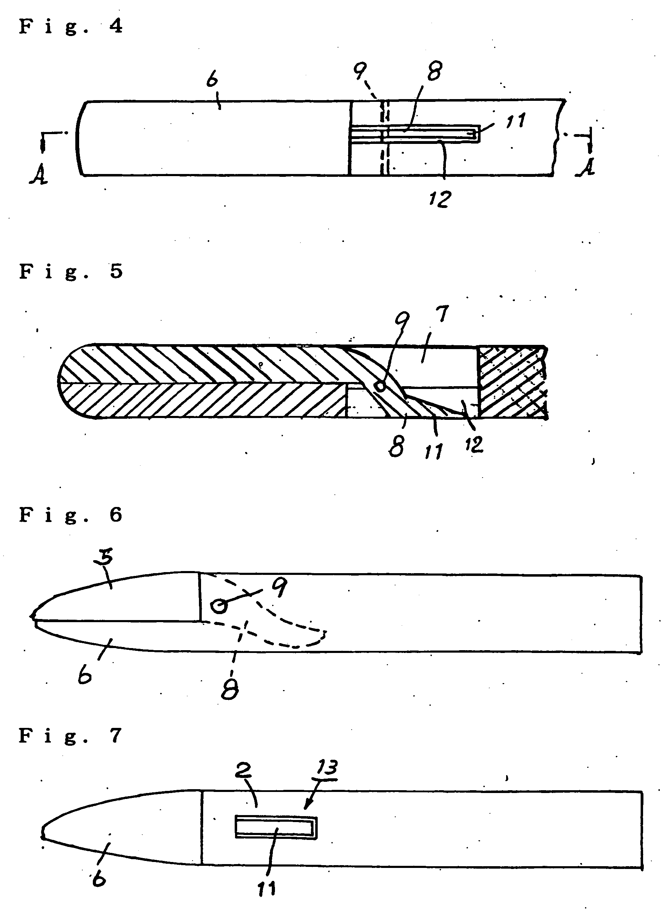

Next, the endoscopic surgical instrument according to the present invention will be described taking the case of having the thread cutting function with reference to Figs. 6 to 9. Except for the thread cutting function, the second embodiment is the same as the first embodiment, so that the different points will be only explained below.

A reference numeral 13 denotes a scissors having the thread cutting function depending on the tubular shaft 2 and the thread locking part 11 of the movable jaw 5. A blade face is formed respectively at the part where the thread locking part 11 of the movable jaw 5 and the tubular shaft 2 can slidably move. When the movable jaw 5 is closed, the thread can be cut between the thread locking part 11 and the tubular shaft. According to the present embodiment, differently from the first embodiment, the thread pulling out gap 12 is not provided between the tubular shaft 2 and the thread locking part 11.

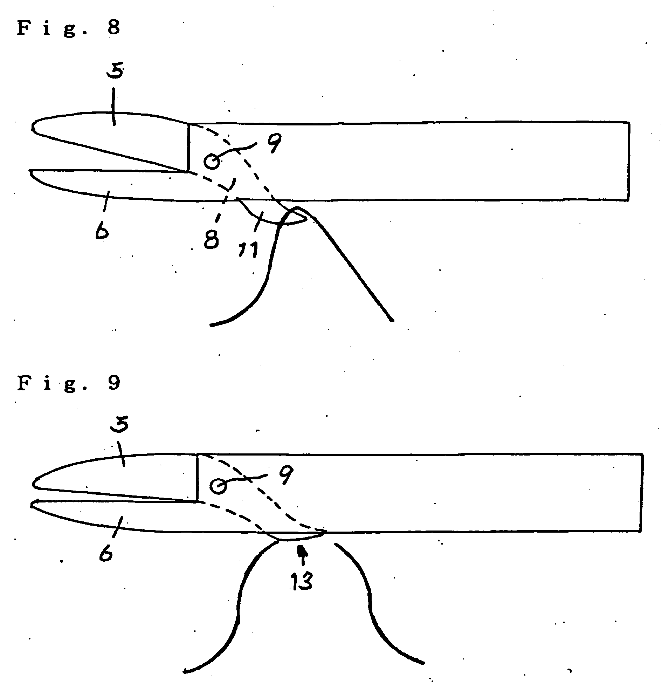

Next, a method of using this endoscopic surgical instrument will be described below. Clipping a needle with a thread between the movable jaw 5 and the fixed jaw 6 of the needle holding part 3 at the front end of the tubular shaft 2, the desired part is sutured. Then, as shown in Fig. 8, after completion of suture, the thread is hitched to the thread locking part 11 and the suture thread is ligated by pulling the endoscopic surgical instrument 1 outside the body. When a knot is formed, as shown in Fig. 9, when the movable jaw 5 is closed, the extra suture thread is cut by the scissors 13 configured by the tubular shaft 2 and the thread locking part 11. Then, the same operation will be repeated at a new suture part.

According to the present invention, with a simple operation, the suture thread can be ligated and in the various endoscopic surgeries, the present invention can be used. In addition, since the operation method of the present invention is simple, a user can learn how to use it simply, so that the present invention can be used at various medical levels.

The features disclosed in the foregoing description, in the claims and/or in the accompanying drawings may, both separately and in any combination thereof, be material for realizing the invention in diverse forms thereof.

## Claims

1. An endoscopic surgical instrument (1) having a needle holding part (3) configured by a movable jaw (5) and fixed jaw (6) at a front end of a tubular shaft (2) that is an insertion part into an abdominal cavity;
wherein the movable jaw (5) is disposed so as to be capable of rotating around the fixed jaw (6) through a pivot shaft (9) due to remote control; the opposite side across the pivot shaft of the movable jaw (5) is formed as a thread locking part (11); when the movable jaw (5) is opened, the thread locking part slightly protrudes outside the tubular shaft (2); and when the movable jaw (5) is closed, the thread locking part (11) is prevented from protruding outside the tubular shaft (2),
**characterized in that** a contact part of the movable jaw (5) and the tubular shaft (2) functions as a scissors (13) so that a suture thread is cut between the thread locking part (11) and the tubular shaft (2) when the movable jaw (5) is closed.

2. The endoscopic surgical instrument (1) according to Claim 1,
wherein an enough gap (12) for the suture thread to freely move is provided between the thread locking part (11) and the tubular shaft (2) with the movable jaw (5) is closed.

## Patentansprüche

1. Ein endoskopisches chirurgisches Instrument (1) mit einem Nadelhalteteil (3), das durch einen beweglichen Schenkel (5) und einen festen Schenkel (6) an dem vorderen Ende eines rohrförmigen Schafts (2), der ein in eine abdominale Höhlung einzusetzendes Teil ist, gebildet ist;
wobei der bewegliche Schenkel (5) derart ausgebildet ist, dass er zu einem Verschwenken zu dem festen Schenkel (6) um einen Schwenkstift (9) aufgrund einer Betätigung von einem entfernten Ort in der Lage ist, die über den Schwenkstift (9) des beweglichen Schenkels (5) gegenüberliegende Seite als ein Fadenverriegelungsteil (11) ausgebildet ist; dann, wenn der bewegliche Schenkel (5) geöffnet wird, der Fadenverriegelungsteil leicht aus dem rohrförmigen Teil (2) vorragt und dann, wenn der bewegliche Schenkel (5) geschlossen ist, der Fadenverriegelungsteil (11) an einem Herausragen aus dem rohrförmigen Schaft (2) gehindert ist,
**dadurch gekennzeichnet, dass** ein Kontaktteil des beweglichen Schenkels (5) und des rohrförmigen Schafts (2) als Schere (13) wirken, so dass ein Nahtfaden zwischen dem Fadenverriegelungsteil (11) und dem Rohrschaft (2) geschnitten wird, wenn der bewegliche Schenkel (5) geschlossen wird.

2. Das endoskopische chirurgische Instrument nach Anspruch 1,
wobei ein für eine freie Bewegung des Nahtfadens ausreichender Spalt (12) zwischen dem Fadenverriegelungsteil (11) und dem Rohrschaft (2) dann, wenn der bewegliche Schenkel, (5) geschlossen ist, vorgesehen ist.

## Revendications

1. Un instrument chirurgical endoscopique (1) ayant une partie de retenue de l'aiguille (3) configurée par une mâchoire mobile (5) et une mâchoire fixe (6) à l'extrémité avant de l'axe tubulaire (2) qui est une pièce d'insertion dans la cavité abdominale ;
dans lequel la mâchoire mobile (5) est disposée de manière à pouvoir se mettre en rotation autour de la mâchoire fixe (6) par le biais d'un axe pivotant (9) grâce à une commande déportée ; le côté opposé de l'axe pivotant de la mâchoire mobile (5) est construit sous la forme d'un élément de verrouillage fileté (11) ; lorsque la mâchoire mobile (5) est ouverte, l'élément de verrouillage fileté dépasse légèrement de l'axe tubulaire (2) ; et lorsque la mâchoire mobile (5) est fermée, le dépassement de l'axe tubulaire (2) de l'élément de verrouillage fileté (11) est bloqué ;
**caractérisé en ce qu'**une partie en contact avec la mâchoire mobile (5) et l'axe tubulaire (2) fonctionne comme des ciseaux (13) de manière à ce qu'un fil de suture soit coupé entre l'élément de verrouillage fileté (11) et l'axe tubulaire (2) lorsque la mâchoire mobile (5) est fermée.

2. Instrument endoscopique chirurgical (1) selon la Revendication 1,
dans lequel un espace suffisant (12) est aménagé entre l'élément de verrouillage fileté (11) et l'axe tubulaire (2) pour que le fil de suture se déplace librement tandis que la mâchoire mobile (5) est fermée.
